# EUROPÄISCHE PATENTANMELDUNG

(11) **EP 1 600 876 A1**
(43) Veröffentlichungstag der Anmeldung: **30.11.2005**
(21) Anmeldenummer: 04012570.0
(22) Anmeldetag: 27.05.2004
(51) Int. Cl.: G06F 19/00

(54) **Elektronische Patientendatenmenge**

(71) Anmelder: Hellmann, Gunther, Dr., 91054 Erlangen (DE)
(72) Erfinder: Hellmann, Gunther, Dr., 91054 Erlangen (DE)

(57) **Zusammenfassung**

Eine elektronisch verbindliche Patientendatenmenge (z.B. Notfalldaten) besteht aus ein oder mehreren Informationseinheiten (z.B. einzelne Notfalldaten) und einer elektronischen Signatur, die über den zuvor genannten Informationseinheiten ermittelt wird. Dabei wird das Hash-Verfahren der Signatur einzeln auf die Informationseinheiten angewendet und anschließend die Folge aus den zusammengesetzten Hash-Werten dem zweiten Schritt der elektronischen Signatur zugeführt. Die elektronisch verbindliche Patientendatenmenge wird auf einem Speichermedium (z.B: Gesundheitskarte) abgelegt oder geändert, wobei auch die Ablage auf Servern möglich ist, deren Ablageadresse über die Gesundheitskarte referenziert wird. Im dem Fall, dass einzelne Informationseinheiten falsch sind, können diese einzeln unkenntlich gemacht werden, indem diese durch deren Hash-Wert ersetzt werden.

Entsprechend der Anzahl der unkenntlich gemachten Informationseinheiten kann die Patientendatenmenge um weitere Datenmengen und dies wiederum ergänzt werden, wobei entsprechend viele Informationseinheiten mit den dann korrigierten Inhalten erzeugt werden. Diese zusätzlichen Informationseinheiten ersetzen somit die unkenntlichen Informationseinheiten.

Die elektronisch verbindliche Patientendatenmenge wird um ein Verzeichnis ergänzt, das separat zu hinterlegen ist, z.B. auf einem getrennt zugreifbarem Bereich. Das Verzeichnis ist durch eine Adresse gekennzeichnet, die auf die abgelegten Daten verweist und eine Liste von Referenzen enthält. Dabei wird jede Person, die sich eine Kopie der Daten nimmt oder Dateneintragung vorgenommen hat, als Referenz gelistet. Damit kann jede dieser Personen über Änderungen informiert werden und die korrigierten Daten erhalten.

## Beschreibung

### Stand der Technik:

Es ist üblich, elektronische Dokumente mit einer elektronischen Signatur zu versehen (Gesetz zur digitalen Signatur. Regulierungsbehörde für Telekommunikation und Post, 2001 oder Kühn, U.: Technische Grundlagen digitaler Signaturverfahren. In Hoeren, T., Schüngel M. [Hrsg.]: Rechtsfragen der digitalen Signatur. Erich Schmidt Verlag, S. 65 -91, 1999), z.B. im speziellen das elektronische Rezept (Debold & Lux: Kosten-Nutzen-Analyse Neue Versichertenkarte und Elektronisches Rezept, Endbericht, Hamburg, 18.5.2001) oder Patientendaten wie z.B. Behandlungsdaten.

Der Grad der Verbindlichkeit der elektronischen Signatur, speziell der der fortgeschrittenen und qualifizierten Signatur, kann durch entsprechend personalisierte Prozessorchipkarten (Signaturkarten) erfüllt werden, indem zumindest der Teilschritt des Verschlüsselns auf dem Chipkartenprozessor der Signaturkarte ausgeführt wird (Managementpapiere des ATG, GVG Köln, 2000; http://atg.gvg-koeln.de/dokumente/21/atg-managementpapier_el-rezept_stand_09-05-2001 _print_copy.pdf).

Zu dem Themenkreis der Gesundheitskarte sind weitere Schriften bekannt, die aber andere Aspekte betreffen:
- Offenlegungsschrift DE 101 64 407 A1,
- Europäische Patentanmeldungsnummer 04009217-3 (betrifft nur die elektronische Verordnung) oder
- Schriften des bit4health-Konsortiums, die unter www dimdi.de publiziert sind.

Zum Thema der Strukturierung von medizinischen Daten gibt es verschiedene Standards und veröffentlichte Spezifikationen (z.B. für Notfalldaten ISO/FDIS 21549-3:2004, Health Information - Patient Healthcare Data - Part 3: Limited Clinical Data).

Nicht selten müssen in der Medizin Daten auf Grund neuer Erkenntnisse oder menschlicher Fehler korrigiert werden. Die Gesundheitskarte dient dabei als Träger der Daten sowie als Referenz für Daten und den mit Daten arbeitenden Akteure.

### Problem:

Der im Patentanspruch 1 angegebenen Erfindung liegt das Problem zugrunde, den von einer Person signierten Datenblock, der sich aus mehreren Teilinformationen zusammensetzen kann, im Falle der Erkenntnis, dass eine oder mehrere Teilinformationen falsch sind, orts- und personenunabhängig unkenntlich zu machen, so dass aber die Signaturprüfung des Datenblocks weiterhin erfolgreich ist.

Der im Patentanspruch 2 angegebenen Erfindung liegt das Problem zugrunde, die unkenntlich gemachten Teilinformationen durch neue Inhalte zu ersetzen ohne gleichzeitig die verbleibenden nicht unkenntlich gemachten Teilinformationen des ursprünglichen Datenblocks signieren und somit bestätigen zu müssen. Diese Teilinformationen liegen nicht zwingend im Verbindlichkeits- und Verantwortungsbereich der korrigierenden Person.

Der im Patentanspruch 3 angegebenen Erfindung liegt das Problem zugrunde, die Personen oder Organisationen, die im Laufe der Zeit die Daten erzeugt haben oder auf die Daten zugegriffen haben und Kopien der Daten in ihre lokalen Systeme übernommen haben, im Falle einer Korrektur der Daten davon in Kenntnis setzen zu können und mit den korrigierte Daten zu versorgen.

### Lösung:

Diese Probleme werden durch die im Patentanspruch 1, 2 und 3 aufgeführten Merkmale gelöst.

### Erreichte Vorteile:

Die mit der Erfindung in Anspruch 1 erzielten Vorteile bestehen insbesondere darin, dass statt einer elektronischen Unterschrift pro Teilinformation die erzeugte elektronische Patientendatenmenge nur einmal in der Gesamtheit elektronisch signiert wird, der zeitkritische Berechnungsschritt durch den Chipkartenprozessor wird nur einmal durchgeführt. Gleichzeitig kann aber zeitlich später eine Teilinformation daraus unkenntlich und nicht wieder herstellbar gemacht werden, ohne dass die restlichen Teilinformationen ihre Verbindlichkeit verlieren.

Ein weiterer Vorteil für Anspruch 1 besteht darin, dass der Vorgang der Unkenntlichmachung von jeder anderen Person durchgeführt werden kann, die ebenfalls über eine Signaturkarte verfügt. Dies ist gerade in den Situation, wo der Urheber der Dokumentation nicht mehr oder nicht verfügbar ist, sehr wichtig, um nicht falsche Information weiterleben zu lassen.

Die mit der Erfindung in Anspruch 2 erzielten Vorteile bestehen insbesondere darin, dass nach der Unkenntlichkeitsmachung einzelner Teilinformationen in Anspruch 1 diese durch korrigierte Inhalte ergänzt werden können. Ein weiterer Vorteil besteht darin, dass die Person, welche dann eine Korrektur durchführt, nicht gezwungen ist, die ursprünglich dokumentierten Daten selber zu signieren, da die Verantwortung nicht in deren Verantwortungs- und Verbindlichkeitsbereich liegt. Ihr Handeln wird dabei durch eine Signatur entsprechend festgehalten.

Ein weiterer Vorteil für Anspruch 2 besteht darin, dass dieses Verfahren auf mehrere Teilinformationen und mehrmals angewendet werden kann. Gleichzeitig können weitere Informationen hinzugefügt werden, ohne einen weiteren Signaturvorgang auszulösen.

Die mit der Erfindung in Anspruch 3 erzielten Vorteile bestehen insbesondere darin, dass nun alle Personen, die sich Kopien der Patientendaten angefertigt haben oder bei der Dokumentation und deren Korrekturen beteiligt waren von jeder Änderung informiert werden können. Dabei kann auch die korrigierte Information zur Verfügung gestellt werden. Es wird somit verhindert, dass sich widersprüchliche Stände der Patientendaten im laufenden Behandlungsprozess bilden und sich falsche Schlussfolgerungen auf der Basis falscher Daten ergeben.

## Patentansprüche

1. Elektronische Patientendatenmenge
• die auf ein Speichermedium (z.B. Gesundheitskarte) aufgebracht, geändert oder darüber referenziert werden oder sind,
• mit einem vorgegebenen elektronischen Signier- und Signaturprüfverfahren bestehend aus
- primärem Hash-Algorithmus und
- anschließender Verschlüsselung unter Einsatz einer Signaturkarte und
• mit den vorgegebenen vertraglichen Rahmenbedingungen insbesondere für Signatur, Datenschutz und des Anwendungsbereiches,
**dadurch gekennzeichnet,**
**dass** die Datenmenge aus
• einer oder mehreren medizinischen, inhaltlich oder zeitlich zusammengehörigen Informationseinheiten (z.B. Notfalldaten),
- die jeweils entweder im Status
"ursprünglich dokumentiert" (im Sprachgebrauch: eingetragen) oder
"unkenntlich" (im Sprachgebrauch: gelöscht) vorliegen,
- im Status "ursprünglich dokumentiert" sind diese durch den für die Informationseinheit gegebenen Datentyp dargestellt und
- im Status "unkenntlich" wird die Informationseinheit durch deren Hash-Wert ersetzt,
• einer elektronischen Signatur,
- die initial über allen Informationseinheiten erstellt wird,
- die für jede Informationseinheit den Hash-Wert separat ermittelt, soweit nicht die einzelne Einheit durch den Hash-Wert bereits ersetzt ist, und
- die die zusammengesetzte Folge aus den ermittelten und ersetzten Hash-Werten für die Verschlüsselung bzw. die Signaturüberprüfung verwendet,
zusammengesetzt sind.

2. Elektronische Patientendatenmenge PDM₁ nach Anspruch 1
**dadurch gekennzeichnet,**
**dass** diese um ein oder mehrere elektronische Patientendatenmengen PDM₂ bis PDM_{n,}
• die sich auf die "unkenntlichen" Informationseinheiten von PDM₁ beziehen,
• die Informationseinheiten im Status "ursprünglich dokumentiert" beinhalten, und
• **dass** die Anzahl der "unkenntlichen" Informationseinheiten von PDM₁ der Anzahl der sich darauf beziehenden "ursprünglich dokumentierten" Informationseinheiten von PDM₂ bis PDM_{n,} welche die unkenntlichen Informationseinheiten von PDM₁ inhaltlich ersetzen, entspricht,
ergänzt sind.

3. Elektronische Patientendatenmengen PDM₁ bis PDMₙ nach Anspruch 1 und 2
**dadurch gekennzeichnet,**
**dass** die Datenmengen durch ein Verzeichnis,
• das mit der Erstellung der elektronischen Datenmenge PDM₁ initial erstellt wird,
• das separat abgelegt ist,
• das aus einer eindeutigen Ablageadresse,
- die sich auf die Gesundheitskarte oder den Patienten der Gesundheitskarte und
- sich auf die elektronische Datenmenge PDM₁ bezieht, und
• einer oder mehreren Referenzen,
- die in eine Adresse der Person oder Organisation
- welche eine oder mehrere der Datenmengen von PDM₁ bis PDMₘ eingetragen hat oder
- sich Kopien der Dateneinheiten PDM₁ bis PDMₘ genommen hat,
- die im Falle der Korrektur von Teildaten über die Korrektur und die korrigierten Inhalte informiert werden können,
umgewandelt werden kann,
zusammengesetzt ist,
ergänzt werden.
